Europäisches Patentamt

European Patent Office

Office européen des brevets

(19)

(11) Publication number: **0 098 718**
**B1**

(12)

# EUROPEAN PATENT SPECIFICATION

(45) Date of publication of patent specification: **07.09.88**

(51) Int. Cl.⁴: **A 61 F 5/448**

(21) Application number: **83303660.1**

(22) Date of filing: **24.06.83**

(54) Improved ostomy device.

(30) Priority: **24.06.82 US 391557**

(43) Date of publication of application:
**18.01.84 Bulletin 84/03**

(45) Publication of the grant of the patent:
**07.09.88 Bulletin 88/36**

(84) Designated Contracting States:
**AT BE CH DE FR GB IT LI LU NL SE**

(56) References cited:
**GB-A-1 571 657**
**GB-A-1 583 027**
**GB-A-2 115 288**
**US-A-4 078 567**

(73) Proprietor: **E.R. Squibb & Sons, Inc.**
**Lawrenceville-Princeton Road**
**Princeton, N.J. 08540 (US)**

(72) Inventor: **Jensen, Ole Roger**
**646 Orangeburg Road**
**River Vale New Jersey (US)**

(74) Representative: **Cook, Anthony John et al**
**D. YOUNG & CO. 10, Staple Inn**
**London, WC1V 7RD (GB)**

Courier Press, Leamington Spa, England.

## Description

The present invention relates to ostomy devices of the type including releaseable interengaging parts adapted to permit detachment of the pouch from the adhesive-backed label and, more particularly, to the structure of the means utilized to mount one of the parts to the label.

Divisional application No. 87101088.0 (EP—A—0235563) contains claims directed to subject-matter disclosed in the present application.

Subsequent to ileostomy, colostomy and similar surgical procedures, it is often necessary for the patient to utilize an ostomy pouch to cover the stoma and collect waste material as it is discharged. Over the years, ostomy pouches of a variety of different sizes, shapes and constructions have been utilized. Various materials and adhesives have been developed to increase the utility and wearability of the pouches.

The basic pouch comprises first and second thin film walls which are sealed, by heat welding or the like, along their periphery to form the contour of the pouch. One wall has an aperture therein designed to receive the stoma. Affixed to the exterior surface of the aperture bearing wall is an adhesive-backed flange or label designed to secure the pouch to the skin surrounding the stoma. The adhesive-backed label has an aperture which aligns with the aperture in the pouch wall. The adhesive-backed label may be affixed to the exterior surface of the pouch in any suitable manner, such as by welding or laminating the adhesive-backed label to the pouch wall. The weld or lamination may take the form of a ring surrounding the aligned apertures.

Over the years, significant advances have been made in the materials employed to construct the labels, particularly in the composition of the base and adhesive coating, such that the labels have become increasingly flexible and porous, permitting the labels to be comfortably worn for extended periods of time. Recent improvements have extended the period during which the labels can be comfortably worn to be longer than normally required for the pouch to fill to capacity with waste material. However, it is still necessary, particularly with new users, to frequently remove the label from the skin to provide access to the skin surrounding the stoma. Removal of the label permits the user to observe and check the condition of the skin surrounding the stoma and, if necessary, to treat same. Thus, in spite of the improvements in label material, frequent removal of the one-piece type device, due to cleaning of the pouch and checking or treatment of the skin surrounding the stoma, is still normally required.

On the other hand, frequent removal of the adhesive-backed label from the skin is to be avoided. The skin surrounding the stoma is often extremely sensitive and may comprise a healing incision or scar tissue. Although the adhesives utilized in the label are formulated to reduce, as much as possible, irritation of the skin when the label is removed, it is preferable to limit the number of times the label must be removed from the skin as much as possible.

One solution to this problem, see patent specification GB—A—1,571,657, is to provide a two-piece ostomy device, wherein the pouch is releasably connectable to the label. The label can then remain affixed to the skin for an extended period of time. The necessity for removing the label each time the pouch is filled is eliminated because the filled pouch can be detached from the label and replaced with a new pouch as often as necessary.

The two-part structure requires a means for releasably connecting the pouch and the label. One particularly successful device takes the form of a pair of annular or ring-like rigid plastic parts — one in the form of a flange, and the other in the form of a groove. The parts are designed to frictionally engage to secure the pouch to the label and, when necessary, to disengage to permit removal of the pouch from the label. The parts are mounted to the pouch and label, respectively. The pouch and label can be connected by simply aligning the parts and pressing same together to cause frictional engagement.

The interengaging part mounted to the pouch surrounds the stoma receiving aperture in the pouch. The part mounted to the label surrounds the aperture in the label. When the interengaging parts are engaged, the apertures automatically correctly align.

When the label is mounted to the skin, the parts are engaged by applying a force on the exterior portion of the pouch over the parts in the direction of the label. This force is of a significant magnitude and is absorbed by the skin beneath the label. However, since the skin under the label may be sensitive, particularly in the time period following the surgical procedure creating the stoma, applying sufficient force to the skin to cause engagement of the parts is often painful and may even be destructive. It is therefore desirable to develop a system which would permit engagement of the parts without the application of significant force on the skin.

It is not possible to simply reduce the amount of force necessary to cause frictional engagement of the parts. This would result in a reduction of the force necessary to detach the pouch from the label, thereby increasing the possibility of accidental detachment. Thus, a different approach to this problem is required.

In published patent application GB—A—2,115,288, published after the date of the present application, a modification is disclosed in which the mounting structure, instead of rigidly retaining the part against the label surface, is made somewhat flexible to permit the user to temporarily lodge his fingers between the part and the surface of the label as the pouch is attached. In this manner, the force applied to interengage the parts is absorbed by the fingers, instead of the sensitive skin surrounding the stoma.

Accordingly, the rigid method of mounting the part to the surface of the label, namely bonding the bottom surface of the part directly to the surface of the label, had to be discarded. Such a structure would not provide the necessary flexibility to permit the fingers to be placed behind the part when the pouch and label were being connected.

Instead of affixing the bottom surface of the part directly to the label in a rigid fashion, the bottom surface of the flange, which extends outwardly of the part, was affixed to the periphery of a circular film member. The member which extends inwardly from the flange toward the center of the part is provided with a central aperture designed to align with the aperture in the label. A ring-like section of the film member, adjacent the aperture, is adapted to be affixed to the surface of the label by welding, lamination, or adhesive. In this manner, the flexibility of the label is somewhat increased and a small space between the part and the surface of the label is provided to permit insertion of the fingers, as the pouch is connected to the label, so as to permit positioning of the fingers to absorb the applied force.

The above-described mounting structure was intended to solve the problem of the application of force to the sensitive skin under the label during the attachment of the pouch. However, other problems inherent in this structure developed. The first problem relates to stress applied to the mounting structure as the fingers are inserted between the part and the surface of the label and to the resulting physical distortion of the interengaging part which makes engagement with the corresponding part difficult. The space available between the part and the surface of the label to accommodate the fingers, even when the surface of the label is flexed, is quite small. For this reason, lodging of the fingers between the part and the surface of the label creates stress on the section of the film member affixed to the label surface at points thereon adjacent the locations where the fingers are inserted. Such stress points tend to dislodge the part from the label because the bond on the section of the film member affixed to the label surface tends to release at the stress points. This structure also results in the lifting of the label from the skin as well as a distortion of the part when the fingers are inserted behind the part.

Another disadvantage of the mounting structure described above relates to the collection of waste material in the space between the surface of the film member and the bottom of the part. Since the film member is bonded to the bottom of the flange which extends outwardly of the part, a pocket is formed between the surface of the film member and the bottom surface of the part. This pocket opens into the interior of the device, toward the aligned stoma receiving openings. Accordingly, waste material moving laterally along the surface of the film member will collect within this pocket where it becomes extremely difficult to remove and provides an area for bacterial growth close to the stoma.

A further disadvantage of the conventional mounting structure is that the pouch surface may be in direct contact with the stoma. While this is normally acceptable, in certain instances, for example, immediately after surgery, it may be desirable to retain the pouch surface at a position spaced from the wound. This is, however, not possible with the structure described above.

Another drawback of conventional detachable pouches, in general, relates to the possibility of the pouch accidentally detaching as it is worn. This is a difficult result to avoid since the amount of force necessary to detach the interengaging parts is determined by the structure thereof and is a function of the amount of force necessary to frictionally engage the parts, which, in turn, is limited, as indicated above, by the amount of force which can be acceptably applied to the skin under the label. Consequently, it is not feasible to alter the structure of the interengaging parts to provide additional security against accidental detachment, unless some complex and costly release mechanism is incorporated into the structure.

Conventional two-piece ostomy devices employ circular interengaging parts. However, there are certain instances where the wound is elongated or the surgical procedure requires the temporary insertion of a plastic tube or glass rod under a section of the intestine. In such circumstances, the circular shape of the interengaging parts may be too restrictive and prevent the use of the device.

In accordance with the primary aspect of the present invention, an improved ostomy device is provided comprising a pouch, an adhesive-backed label, and means for releasably connecting the pouch and the label, the connecting means comprising first and second interengaging parts, means for mounting the first part to the pouch and means for mounting the second part to the label, at a normal position relative to the surface of the label, the second part mounting means comprising a section, spaced from the second part, adapted to be affixed to the surface of the label, and an expandable means, interposed between the section and the second part, for facilitating displacement of the second part relative to its normal position.

The second part mounting means preferably comprises a circular or oval member composed of thin material, preferably plastic, of sufficient strength and thickness to maintain the pouch in a position proximate the surface of the label, even when the pouch is full of waste material. The section affixed to the label surface preferably includes a ring-shaped or oval weld or laminate.

The expandable means preferably comprises one or more accordion-like folds in the member. The expandable means may extend in a direction substantially parallel to the surface of the member or may extend in a direction substantially perpendicular to the surface of the member.

The label is provided with an aperture therein. The second part mounting means is provided with a central aperture, situated to align with the label aperture. The section of the second part mounting means affixed to the label preferably surrounds the aligned aperture.

The second part mounting means preferably comprises a second section extending between and joining the expandable means and the part. The second section is preferably sealed to the part along the edge of the part between the inside surface and bottom surface thereof. In this manner, space between the part and the second section is eliminated and accumulation of waste material between the part and the mounting means is prevented.

In accordance with a preferred arrangement according to the present invention, means are provided, extending from the first part, which are effective to cover the portion of the second part mounting means proximate to the second part.

The second part mounting means comprises expandable means. The portion of the second part mounting means proximate to the second part and protected by the cover means preferably comprises the expandable means.

The second part mounting means preferably comprises a section adapted to be affixed to the surface of the label. The portion of the second part mounting means proximate to the second part and protected by the cover means preferably comprises the portion of the second part mounting means between the section affixed to the surface of the label and the second part.

The cover means preferably comprises an element having a body portion and an unattached end. The body portion is adapted to be situated in registration with the portion of the second part mounting means proximate the second part, when the parts interengage. The unattached end of the covering element is adapted to contact the surface of the second part mounting means when the parts interengage.

The first interengaging part preferably comprises a ring-shaped or oval engaging part. The cover means preferably comprises a flange-like element extending from the inner surface of the engaging part towards the center thereof.

In accordance with another aspect of the present invention, means are provided for retaining the expandable means in the expanded condition.

Retaining the expandable means in the expanded condition maintains the pouch surface at a location spaced from the wound. Preferably, the retaining means takes the form of a detachable ring formed of elastic material to permit same to be inserted underneath the member, preferably that portion of the member in alignment with the connecting means mounted thereon.

In accordance with yet another aspect of the present invention, secondary connecting means are provided for releasably connecting the pouch and the label in addition to the primary connection which is provided by said first and second inter-connecting parts.

The secondary connecting means preferably comprises a protrusion and aperture structure or a hook and eye structure. The secondary connecting means is preferably located proximate the top of the pouch primary connecting means and the label. In this manner, should the primary connecting means become accidentally detached, the secondary connecting means will prevent the pouch from detaching from the label. The secondary connecting means also serves to aid in supporting the pouch as the pouch becomes heavier due to the accumulation of waste material therein.

To these and such other objects which may hereinafter appear, the present invention relates to an improved ostomy device, as set forth in detail in the following specification and recited in the annexed claims, taken together with the accompanying drawings, wherein like numerals refer to like parts, and in which:

Figure 1 is an exploded isometric view of a prior art ostomy device of the type shown in GB—A—1,571,657 and which includes a label, a pouch, and means for releasably connecting the label and pouch;

Figure 2 is a cross-sectional view of a similar ostomy device to that shown in Figure 1, but modified according to the principles of the aforesaid later published GB—A—2,115,288;

Figure 3 is an isometric view of a first preferred embodiment of the label portion of the improved ostomy device of the present invention;

Figure 4 is a cross sectional view of the embodiment of Figure 3 but showing the complete device;

Figure 5 is a cross-sectional view similar to Figure 4 and illustrating the manner in which the parts of the first embodiment are engaged;

Figure 6 is a cross-sectional view of a second preferred embodiment of the expandable means used in a device according to the present invention;

Figure 7 is a cross-sectional view of a third preferred embodiment of the expandable means used in a device according to the present invention;

Figure 8 is an isometric view showing a first preferred embodiment of the secondary connecting parts optionally used in the present invention;

Figure 9 is an isometric view showing a second preferred embodiment of the secondary connecting parts optionally used in the present invention;

Figure 10 is a front view of a label portion of a device according to the present invention and incorporating a fourth preferred embodiment of the expandable means;

Figure 11 is a cross-sectional view of the embodiment illustrated in Figure 10, showing the manner in which the parts are interengaged;

Figure 12 is a cross-sectional view of the embodiment illustrated in Figure 10, in the assembled condition;

Figure 13 is a front view of an embodiment of the present invention, wherein the interengaging parts are oval;

Figure 14 is a cross-sectional view of a device according to the present invention and incorporating a first preferred embodiment of an expansion retaining means.

Figure 15 is a horizontal section taken along line 15—15 of Figure 14;

Figure 16 is a cross-sectional view of a device according to the present invention and incorporating a second preferred embodiment of an expansion retaining means; and

Figure 17 is a cross-sectional view of a device according to the present invention and incorporating a third preferred embodiment of an expansion retaining means.

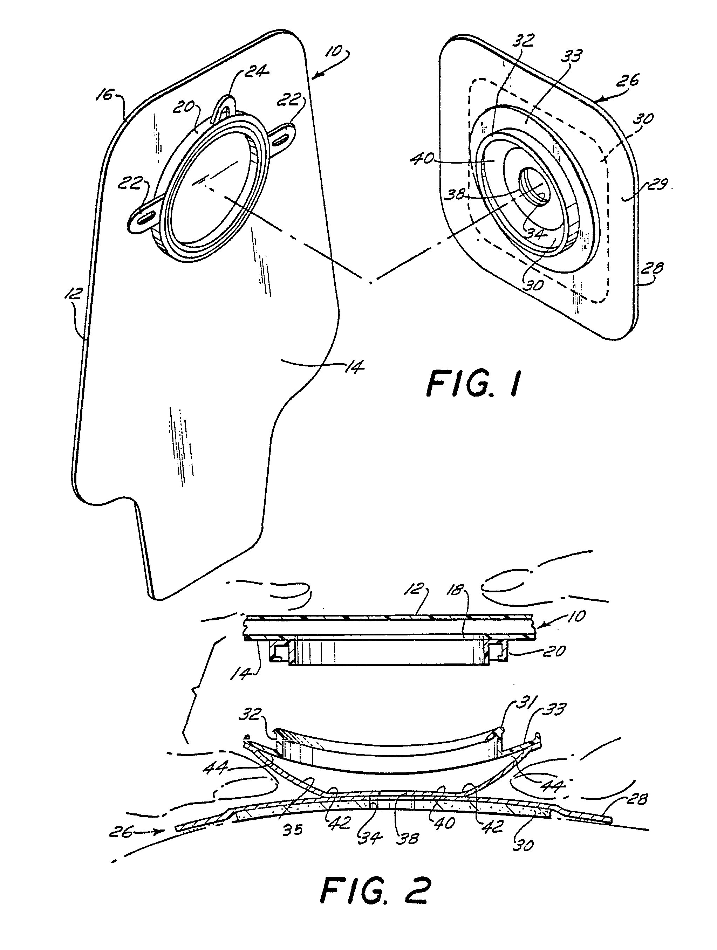

Fig. 1 illustrates a commercially available ostomy device of the type disclosed in GB—A—1,571,657 and incorporating a detachable pouch. The pouch, generally designated 10, comprises a first or outer wall 12 and a second or inner wall 14, both composed of a thin, moisture-impermeable, odor-proof, thermo-plastic film or laminate. The interior surfaces of walls 12 and 14 are sealed along the periphery 16 thereof, so as to form the contour of the pouch. Wall 14 is provided with a stoma receiving aperture 18.

Surrounding aperture 18 is an annular plastic part 20, preferably in the form of a ring-shaped groove, which forms one of the interconnecting parts. The rear surface of part 20 is affixed to the exterior surface of wall 14 in any appropriate manner, such as welding or by a layer of adhesive or the like. Extending outwardly from the side of part 20, in opposite directions, are a pair of belt-receiving elements 22 which can be utilized to connect part 20 and, thus, pouch 10 to a belt designed to extend around the body. Also extending from the side of part 20, near the top thereof, is a tab 24 designed to facilitate grasping of part 20.

The conventional ostomy device illustrated in Fig. 1 also includes an adhesive-backed label, generally designated 26. The purpose of the adhesive-backed label 26 is to affix the device to the skin of the wearer. The adhesive-backed label 26 must serve to securely retain the device to the skin surrounding the stoma for an extended period of time.

Adhesive-backed label 26 comprises a base 28 which can be formed of porous material and an adhesive layer 30 situated on the rear surface of base 28.

To the front surface 29 of base 28 is mounted the second interengaging part 32, which preferably takes the form of an annular or ring-like protrusion 31 and an outwardly extending flange 33. Protrusion 31 is fashioned to frictionally engage groove-like part 20. Part 32 is mounted on label 26 at a point surrounding an aperture 34 in the label. Aperture 34 in label 26 is designated to align with aperture 18 in pouch 10 when parts 20 and 32 are frictionally engaged.

As discussed previously, part 32 is, in accordance with the teachings of GB—A—1,571,657 affixed directly to the surface 29 of base 28. However, this does not permit the user to insert his fingers between part 32 and label 26, so as to absorb the force applied to interengage parts 20 and 32.

Consequently, the alternative mounting method shown in Figure 2 has been proposed in the aforesaid later published GB—A—2,115,288. In this modification part 32 is mounted to base 28 of label 26 through the use of a circular-shaped thin film member 35. Film 35 is provided with an aperture 38, at the central portion thereof. Aperture 38 aligns with aperture 34 in label 26. Film 35 is affixed to base 28 by means of a ring-shaped weld, laminate, or adhesive section 40 surrounding, but spaced a short distance from, aperture 38. Film 35 extends outwardly from section 40 and is affixed to the exterior edge of the bottom surface of flange 33.

This mounting structure increases the flexibility of label 26 to a small degree as compared to the construction where part 32 is affixed directly to the label surface. In addition, it permits part 32 to be displaced relative to the surface 29 of base 28, to a small extent, to permit the fingers of the user to be situated between label 26 and part 32 so as to absorb the force applied when part 20 is caused to frictionally engage part 32.

However, as indicated above, the structure disclosed in Figure 2 has two major drawbacks. When the fingers are inserted behind part 32, as shown in Figure 2, substantial stress is concentrated at points 42 near the exterior of section 40, adjacent the position of the fingers. These stress points 42 tend to disrupt the integrity of section 40 and may cause film member 35 to detach from base 28.

In addition, affixing film member 35 to the exterior edge of bottom surface of flange 33 creates a pocket 44 between the upper surface of film member 35 and lower surface of flange 33, into which waste material may collect. Because of the position and size of pocket 44, it is extremely difficult to remove the collected waste material therein. Consequently, the label may become unusable long before it would normally be necessary to remove the label from the skin.

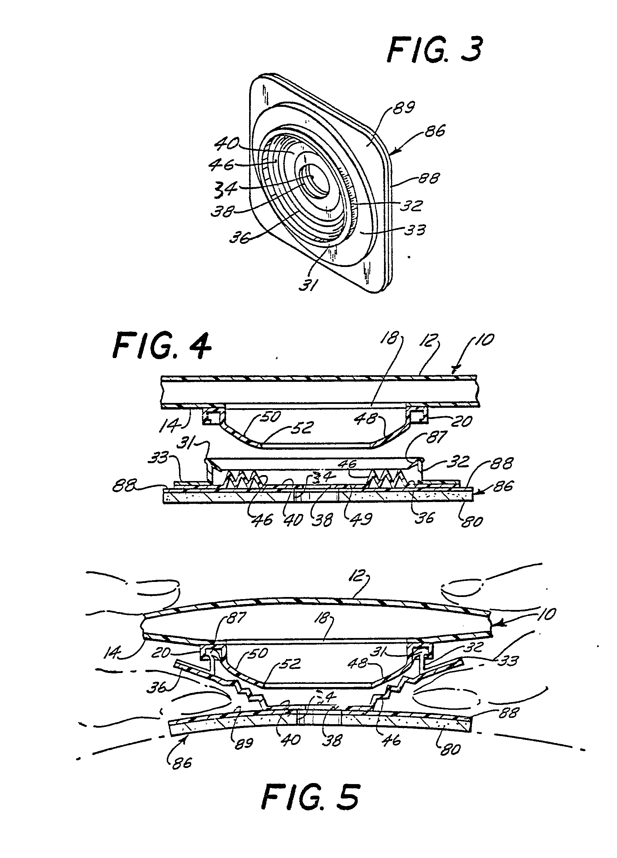

Both of these disadvantages associated with the mounting structure, illustrated in Fig. 2, are eliminated by the structure of the present invention. Figs. 3, 4, and 5 illustrate the structure and function of the first preferred embodiment of the present invention, which is designed to overcome the defects noted above. All parts of the device of the present invention illustrated in Figs. 3, 4, and 5 are identical to those illustrated in Figs. 1 and 2, with the exceptions noted below. For this reason, a detailed description of the previously described parts and the function of same is omitted.

Adhesive-backed label 86 comprises a base 88 which is preferably a thin film of polymeric material such as polyethylene and an adhesive layer 80 situated on the rear surface of base 88. Adhesive layer 80 is preferably formed as a homogeneous blend of one or more pressure-sensitive viscous or elastomeric materials having intermittently dispersed therein one or more water-soluble or swellable hydrocolloid gums and may also include one or more thermoplastic elastomers and/or one or more swellable cohesive strengthening agents.

In general, the defects of the structure illustrated in Fig. 1 are eliminated in the present invention by employing a member 36 to mount part 32 to base 88 of label 86 which includes expandable means situated between welded section 40 and the portion of member 36 affixed to part 32. Member 36 is preferably made of plastic or similar material of sufficient strength and thickness to maintain the pouch in a normal position proximate the surface of label 86, even when the pouch is filled with waste material. The structure of member 36, and particularly the expandable means, permits a greater degree of flexibility of the label, facilitates displacement of part 32 from its normal positions proximate the surface of label 86 during mounting of the pouch, and eliminates any stress points resulting from the displacement. In addition, by affixing member 36 to the bottom of part 32, adjacent the interior edge thereof, pocket 44 is eliminated and, as a result, the collection of waste material beneath part 32 is prevented.

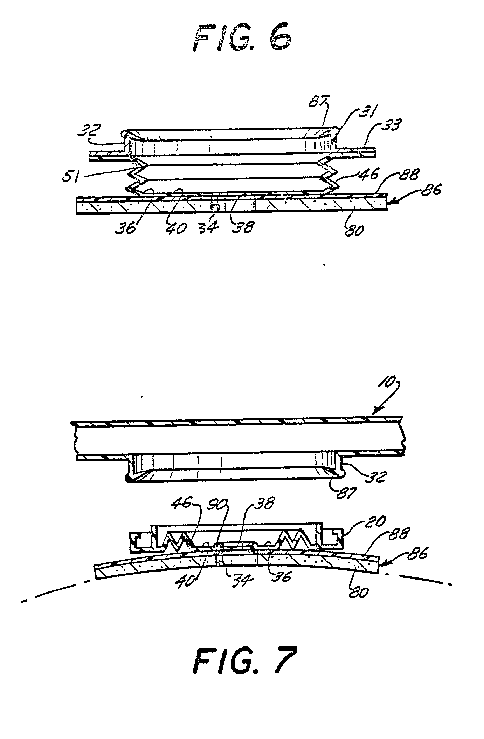

Fig. 3 illustrates the structure of the label of the present invention. Figs. 4 and 5 illustrate the manner in which the new label structure facilitates engagement of parts 20 and 32 through the use of the expandable means which forms a portion of member 36. Figs. 4 and 5 also illustrate the cover means associated with the part mounted on the pouch and the manner in which the cover means protects the expandable means when the parts are engaged. The expandable means preferably comprises one or more accordion-like folds 46 (three are illustrated in Figs. 3, 4, and 5) formed in member 36 at a location between section 40 and the portion of member 36 to which part 32 is affixed. Accordion-like folds 46 facilitate the displacement of part 32 relative to surface 89 by permitting member 36 to expand when the fingers are inserted between the part and the label, and, as a result, eliminate stress on points along section 40 such that the integrity of section 40 is not likely to be disrupted in normal usage.

It should be noted that, in the preferred embodiment of applicant's invention, member 36 is affixed to the bottom surface of flange 33 at a point spaced from accordion-like folds 46, through the use of an adhesive layer or the like. The upper surface of member 36 is adjacent the interior edge of part 32. This manner of affixation eliminates pocket 44 present in the conventional mounting structure between part 32 and member 36. Thus, with the structure of the present invention, it is not possible for any waste material to collect under part 32.

It should also be appreciated that flange 33, extending outwardly of protrusion 31, can be eliminated, if desired. In that case, member 36 would be affixed to the rear surface of protrusion 31, adjacent to the interior side thereof. This structure also eliminates the possibility of waste build-up under part 32.

Another aspect of applicant's invention is also illustrated in Figs. 4 and 5. Part 20, affixed to wall 14 of pouch 10, is provided with a flange-like cover element 48 which is preferably mounted to and extends from the edge or side of part 20. Element 48 comprises a body portion 50 and rim 52. Body portion 50 is designed to be in registration with the accordion-like folds 46 on member 36, when parts 20 and 32 interengage. The purpose of body portion 50 is to cover and protect accordion-like folds 46 from waste material, which may move laterally along member 36 from the stoma receiving aperture towards part 32. The ring 52 of element 48 is biased, due to the resiliency of body 50, toward the section 49 of the surface of member 36, between folds 46 and aperture 38. In this manner, element 48 tends to seal the portion of member 36 containing folds 46 from any waste material.

Fig. 6 illustrates the second preferred embodiment of mounting means of the present invention. This embodiment is identical in all respects to the embodiment shown in Fig. 4, except that accordion-like folds 46, instead of normally extending in a direction substantially parallel to surface 89, normally extend in a direction substantially perpendicular to surface 89. In this case, the last or uppermost fold 51 is directly affixed to the bottom of protrusion 31 of part 32, such that no waste material can collect under part 32. Here again, flange 33 may be eliminated, if desired.

It will now be appreciated that accordion-like folds 46 may expand in a direction either parallel to or perpendicular to surface 89. The only limitation on the number and structure of the accordion-like folds is that member 36 must have sufficient strength to prevent substantial expansion during normal use of the device. That is, member 36 must have sufficient strength to withstand substantial expansion caused by the weight of the pouch, and thus maintain part 32 proximate surface 89, even when the pouch is filled to capacity.

Fig. 7 illustrates a third preferred embodiment of the present invention which differs from the previously described embodiments in two important aspects. As seen in Fig. 7, the positions of interengaging parts 20 and 32 have been interchanged such that part 20, which comprises the ring-like groove or channel, is mounted to label 86 instead of pouch 10, and part 32, which comprises the ring-like protrusion or flange, is mounted to pouch 10 instead of label 86. This interchange of the positions of interengaging parts 20 and 32 does not alter the function of the device but does provide a significant manufacturing advantage.

In the first embodiment, with part 32 mounted to label 86, it is not possible to injection mold part 32 and mounting member 36 in a single operation. This is because in order to properly form the lip 87, which extends inwardly from flange 31 and enhances the frictional engagement with the groove or channel of part 20, a mold part must be provided behind the lip as the lip is formed. Accordingly, part 32 and member 36 cannot be formed in a single operation, but must be formed separately and thereafter joined together. However, part 20 and member 36 can be formed integrally, in a single injection molding operation, providing a significant manufacturing advantage.

The second structural difference relates to the addition of a bead 90 of material provided in member 36 at the interior edge of section 40 which surrounds the portion of base 88 where the stoma receiving aperture is situated. Base 88 is normally supplied by the manufacturer with a very small central aperture which is enlarged to the appropriate size before positioning the label portion around the stoma. If the aperture in base 88 is enlarged all the way to the interior edge of section 40 of member 36, the exposed edge of section 40 may be sharp enough to cut the side of the stoma as the device is worn. This problem is eliminated by creating a bead 90 of material, at the interior edge of section 40 of member 36. Because of the rounded structure of bead 90, the possibility of a sharp edge resulting from enlargement of the central aperture is eliminated. Moreover, bead 90 provides a convenient visual means of determining the maximum size to which the stoma receiving aperture may be enlarged.

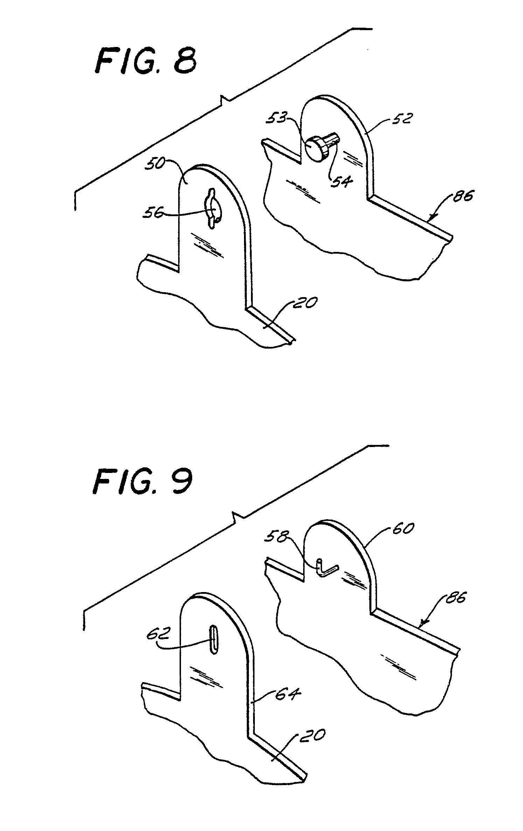

Figs. 8 and 9, respectively, illustrate two preferred embodiments of an optional feature of the present invention which relates to the use of auxiliary connecting means. Because the force which may be applied to cause frictional engagement of the interengaging parts is limited, it is difficult to prevent the occasional accidental detachment of the interengaging parts. For this reason, devices according to the present invention are preferably provided with auxiliary interengaging parts which engage independently of the primary interengaging parts and provide an extra measure of security against the accidental detachment of the pouch from the label. The auxiliary interengaging parts also aid to support the weight of the pouch when filled.

As illustrated in Fig. 8, the auxiliary interengaging parts comprise a pair of aligned tabs 50, 52 extending from the top of pouch primary interengaging part 20 and label 86, respectively. Secondary interengaging part 52 is provided with a protrusion 54 extending from one surface thereof. Protrusion 54 is preferably provided with an enlarged tip 53. The other part 50 is provided with an aperture 56 designed to frictionally receive protrusion 54 therein. Before or after the primary interengaging parts 20 and 32 are frictionally engaged, the user places her fingers on either side of the aligned tab pair 50, 52 and forces the protrusion 54 into the aperture 56, such that enlarged tip 53 is situated adjacent the opposite surface of tab 50, so as to releasably connect the tabs. When the pouch is removed from the label, the process is reversed, with protrusion 54 being pushed out from the aperture 56 to release the tabs.

Fig. 9 shows a modification of this aspect of the present invention wherein the protrusion and aperture combination is replaced with a hook and eye configuration. In this case, hook 58 extends from one tab 60 and is designed to engage aperture 62 on the other tab 64 when primary interengaging parts 20 and 32 are connected.

Whichever version of the secondary interengaging parts is utilized, it is preferable that the tabs extend upwardly from part 20 and label 86 at the tops thereof. In this position, the secondary interengaging parts will retain the pouch on the label, even if parts 20 and 32 become disconnected.

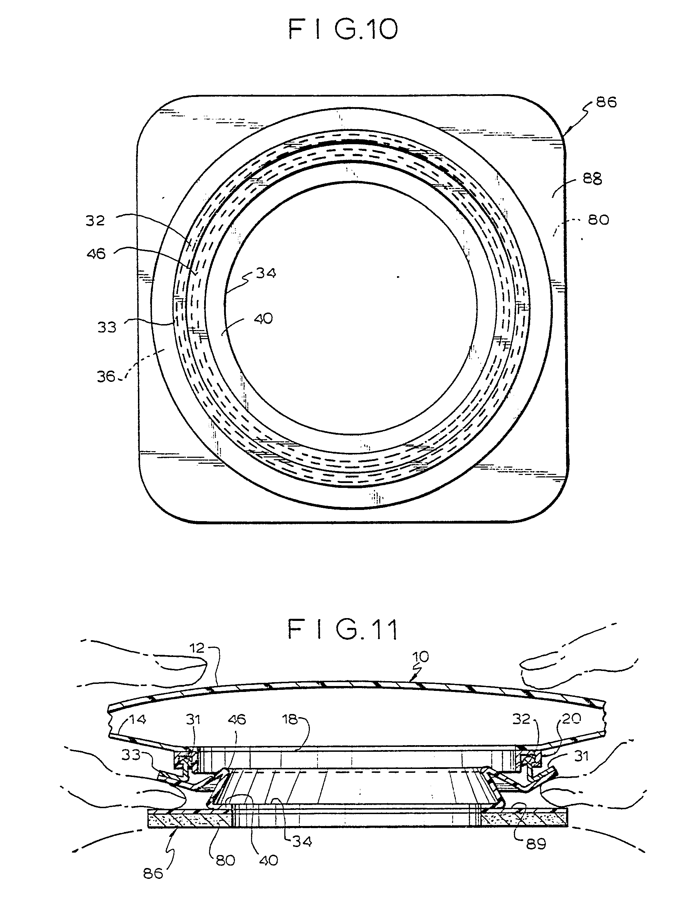

Figs. 10, 11, and 12 illustrate a modification of the present invention wherein the expandable means includes only a single accordion-like fold 46. This configuration has several advantages over the multi-fold structure illustrated in Figs. 3 through 6, in that the single fold permits a larger stoma receiving aperture 34 and tends to expand more easily during assembly with the pouch. Moreover, the device is easier to clean with only a single fold 46, as compared to the multi-fold version.

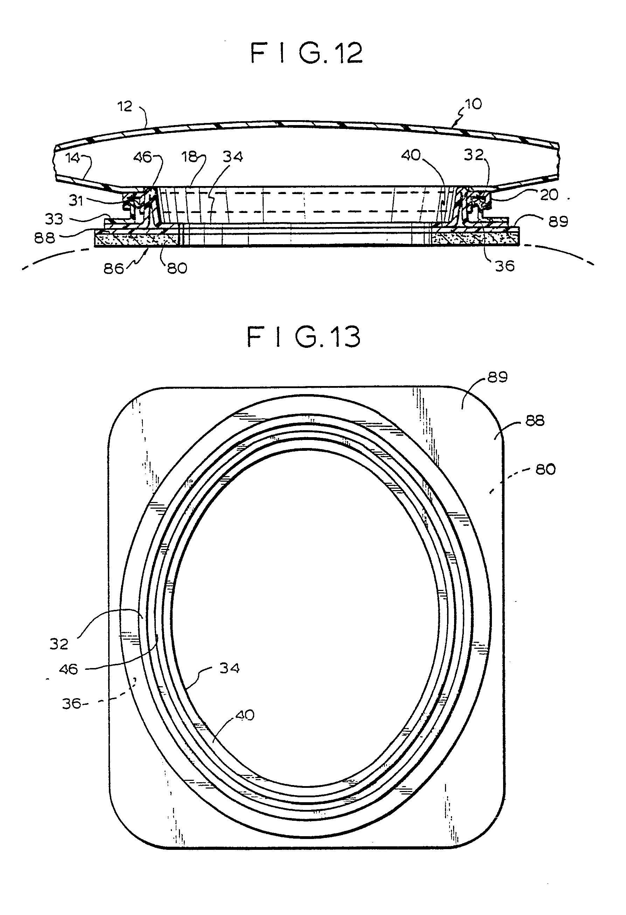

Another advantage of the single fold embodiment is illustrated in Fig. 12. Once the pouch piece is connected to the label piece, and the single fold 46 is in the non-expanded condition, the interior vertical surface of the fold acts as a barrier to prevent waste material from lodging between fold surface facing part 32 and the surface of part 32. Thus, no element 48 on part 20 is required to prevent lodging of waste material between the fold and part 32.

Fig. 13 illustrates a modification of the shape of the interengaging parts 20 and 32 where the parts are elongated, preferably oval, as opposed to circular, as illustrated in Fig. 10. This configuration permits the use of the device with elongated wounds and after certain types of surgery which requires an unobstructed elongated area around the wound.

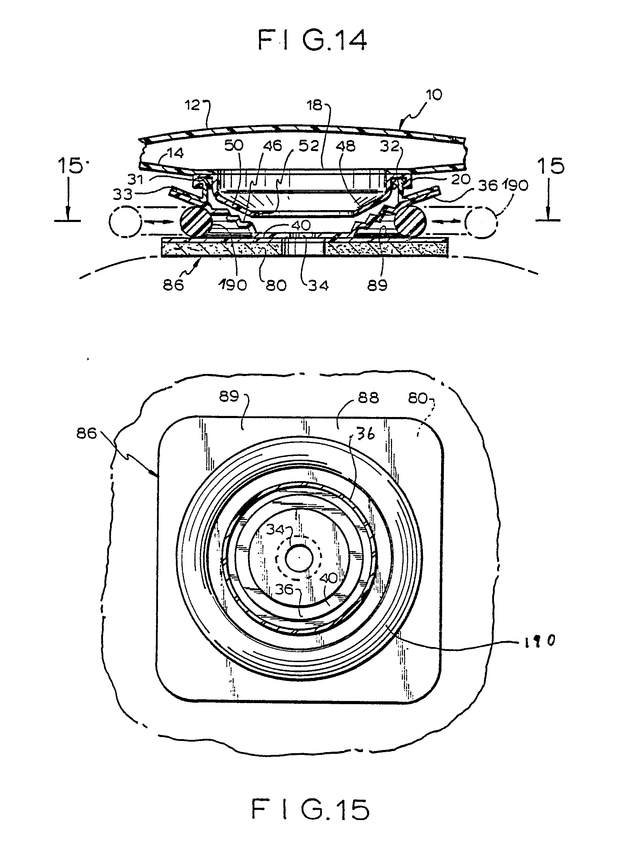

Figs. 14—17 illustrate the use of a means for retaining the expandable means 46 in the expanded condition. Retaining the folds 46 in the expanded condition will maintain the surface of the pouch at a location remote from the stoma, which may be required immediately after surgery.

The retaining means preferably comprises a ring-like element 190 composed of elastic material, such as closed cell foam or the like which can be expanded to fit over the outwardly extending flange 33 of part 32, and then released such that it lodges beneath member 36, preferably below part 32. Thus, element 190 will then retain the folds 46 in the expanded condition.

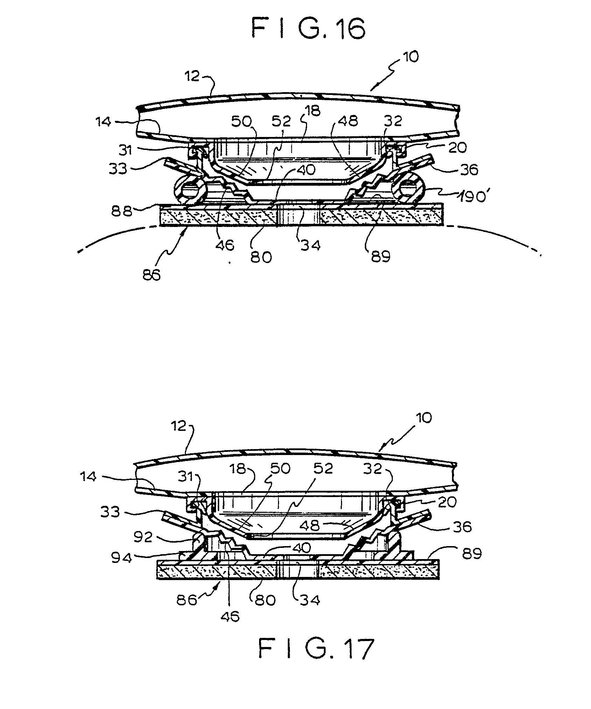

Element 190 can take various forms and may be detachable or simply pivotably connected to the label, by an articulated or flexible joint, in which case, it can be pivoted to a remote position when not in use. Moreover, element 190 may have a solid cross-section and be in the form of a detachable "O"-ring, as illustrated in Figs. 14 and 15, a detachable "O"-ring of hollow cross-section, as illustrated in Fig. 16 as 190', or be a detachable ring-like member 190'' with an upstanding part 92 and an enlarged base 94, as illustrated in Fig. 17.

It should be understood that when an expansion retaining means is utilized, it is possible to convert the ostomy device into an irrigation and/or drain device by the addition of inlet and/or outlet ports in appropriate locations. This provides the device of the present invention with versatility not possible in conventional devices of this type.

It will now be appreciated that the present invention relates to an ostomy device with a label and a detachable pouch wherein the structure employed to mount the interengageable part to the label increases the flexibility of the label and facilitates displacement of the part relative to the surface of the label without creating undue stress on the section of the mounting member affixed to the surface of the label. In addition, the pocket or gap between the mounting member and the interengaging part is eliminated such that the accumulation of waste material beneath the interengaging part is prevented.

The displaceability of the interengaging part relative to the surface of the label is facilitated by incorporating expandable means, in the form of one or more accordion-like folds, in the mounting member between the part and the section of the mounting member affixed to the label. The accordion-like folds can extend in a direction parallel to or perpendicular to the surface of the label. The part affixed to the pouch is preferably provided with a flange-like cover element designed to align with and protect the accordion-like folds from waste, when the interengaging parts are connected.

Accumulation of waste material beneath the interengaging part mounted to the label is prevented by affixing the under surface of the part to the mounting member adjacent the interior edge of the part. Thus, no space between the part and member is present.

Accidental detachment of the pouch from the label is prevented by providing secondary connecting means in the form of auxiliary interengaging parts. The auxiliary interengaging parts preferably extend from the top of the pouch primary interengaging part and label, and preferably take the form of interlocking tabs including a protrusion and aperture or hook and eye configuration.

Through the use of expansion retaining means, the expansion of the accordion-like fold can be maintained in order to keep the pouch at a location remote from the stoma. Moreover, for wounds which require an unobstructed elongated area, the interengaging parts may be made oval instead of round.

While only a limited number of preferred embodiments of the present invention have been disclosed herein for purposes of illustration, it is obvious that many variations and modifications could be made thereto.

## Claims

1. An ostomy device comprising a pouch (12), an adhesive-backed label (26) and means (20, 32) for releasably connecting said pouch and label, said connecting means comprising first (20) and second (32) interengaging parts, means for mounting said first part to said pouch and means for mounting said second part to said label, at a normal position relative to the surface of said label, said second part mounting means comprising a section (40), spaced from said second part, adapted to be affixed to the surface of said label, characterised by expandable means (46), interposed between said section (40) and said second part (32), for facilitating displacement of said second part (32) relative to said normal position.

2. The device of claim 1, wherein said second part mounting means comprises a member and wherein said expandable means (46) comprises one or more accordion-like folds in said member.

3. The device of claim 1, wherein said expandable means (46) extends in a direction substantially parallel to said surface of said label (26).

4. The device of claim 1, wherein said expandable means extends in a direction substantially perpendicular to said surface of said label (26).

5. The device of claim 1, wherein said second part mounting means further comprises a second section (33) also herein called a flange extending between and joining said expandable means (46) and said second part (32).

6. The device of claim 5, wherein said second section is affixed to said second part (32) at a point on the under surface of said part adjacent the side of said part.

7. The device of claim 1, wherein said first part (20) further comprises means (48) adapted to cover said expandable means (46) when said first and second parts are interengaged.

8. The device of claim 7, wherein said cover means comprises a flange-like element (50) extending from said first part.

9. The device of claim 8, wherein said element comprises a body portion and an unattached end (52), said body portion (50) being adapted to be situated in registration with said expandable means (46) and said end being adapted to contact the surface of said second part mounting means, when said parts interengage.

10. The device of claim 9, wherein said body portion (50) is resilient and biases said end (52) into contact with said surface, when said parts are interengaged.

11. The device of claim 1, further comprising second means for releasably connecting said pouch and said label, said second connecting means comprising first and second auxiliary interengaging parts (50, 52), means for mounting one (50) of said auxiliary interengaging parts to said pouch and means for mounting the other of said auxiliary interengaging parts (52) to said label (86).

12. The device of claim 11, wherein said auxiliary interengaging parts comprise a hook (58) and eye (62).

13. The device of claim 11, wherein said auxiliary interengaging parts comprise a protrusion (53) and an aperture (56).

14. The device of claim 1, wherein said interengaging parts comprise a protrusion and groove.

15. The device of claim 1, wherein said interengaging parts are annular.

16. The device of claim 1, wherein said interengaging parts comprise a ring-shaped protrusion and a ring-shaped groove.

17. The device of claim 1, wherein said first part comprises a ring-shaped protrusion and said second part comprises a ring-shaped groove.

18. The device of claim 1, wherein said first part comprises a ring-shaped groove and said second part comprises a ring-shaped protrusion.

19. The device of claim 1, further comprising a bead situated along the interior edge of said section of said second part mounting means.

20. The device of claim 1, wherein the interior edge of said section of said second part mounting means is rounded.

21. The device of claim 1, further comprising means (190) for retaining said expandable means in the expanded condition.

22. The device of claim 21, wherein said retaining means comprises an element interposable between said second part mounting means and said label.

23. The device of Claim 21, wherein said retaining means is detachable.

24. The device of Claim 21, wherein said retaining means comprises a ring-like element.

25. The device of Claim 21, wherein said retaining means is composed of elastic material.

26. The device of Claim 21, wherein said retaining means is composed of closed cell foam.

27. The device of Claim 21, wherein said retaining means has a substantially circular cross-section.

28. The device of Claim 21, wherein said retaining means comprises an upstanding section and a base section.

29. The device of Claim 1, wherein said interengaging parts have an elongated configuration.

30. The device of Claim 1, wherein said interengaging parts have a substantially oval configuration.

**Patentansprüche**

1. Ostomievorrichtung, die einen Beutel (12), ein klebstoffhinterlegtes Plättchen (26) und eine Einrichtung (20, 32) zum lösbaren Verbinden des Beutels und des Plättchens aufweist, wobei die Verbindungseinrichtung erste (20) und zweite (32) ineinandergreifende Teile, eine Einrichtung zum Anbringen des ersten Teils an dem Beutel und eine Einrichtung zum Anbringen des zweiten Teils an dem Plättchen an einer vorbestimmten Stellung relativ zur Fläche des Plättchens aufweist, und wobei die Anbringungseinrichtung für den zweiten Teil einen Abschnitt (40) aufweist, der einen Abstand von dem zweiten Teil hat und derart ausgelegt ist, daß er an der Fläche des Plättchens befestigbar ist, gekennzeichnet durch eine dehnbare Einrichtung (46), die zwischen dem Abschnitt (40) und dem zweiten Teil (32) zum leichten Verschieben des zweiten Teils (32) relativ zu der Grundstellung angeordnet ist.

2. Ostomievorrichtung nach Anspruch 1, bei der die Anbringungseinrichtung für das zweite Teil ein Element aufweist, und bei der die dehnbare Einrichtung (46) eine oder mehrere ziehharmonikaähnliche Faltungen in dem Element aufweist.

3. Ostomievorrichtung nach Anspruch 1, bei der die dehnbare Einrichtung (46) in eine Richtung im wesentlichen parallel zu der Fläche des Plättchens (26) verläuft.

4. Ostomievorrichtung nach Anspruch 1, bei der die dehnbare Einrichtung in einer Richtung im wesentlichen senkrecht zur Fläche des Plättchens (26) verläuft.

5. Ostomievorrichtung nach Anspruch 1, bei der die Anbringungseinrichtung für das zweite Teil ferner einen zweiten Abschnitt (33), der auch als ein Flansch bezeichnet wird, aufweist, der sich zwischen der dehnbaren Einrichtung (46) und dem zweiten Teil (32) erstreckt und diese verbindet.

6. Ostomievorrichtung nach Anspruch 5, bei der der zweite Abschnitt an dem zweiten Teil (32) an einer Stelle auf der unteren Fläche des Teils in der Nähe der Seite des Teils befestigt ist.

7. Ostomievorrichtung nach Anspruch 1, bei der das erste Teil (20) ferner eine Einrichtung (48) aufweist, die derart ausgelegt ist, daß sie die dehnbare Einrichtung (46) bedeckt, wenn die ersten und zweiten Teile ineinandergreifen.

8. Ostomievorrichtung nach Anspruch 7, bei der die Abdeckeinrichtung ein flanschähnliches Element (50) aufweist, das sich von dem ersten Teil weg erstreckt.

9. Ostomievorrichtung nach Anspruch 8, bei der das Element einen Körperabschnitt und ein nicht angebrachtes Ende (52) aufweist, der Körperabschnitt (50) derart ausgelegt ist, daß er in ausgerichteter Lage zu der dehnbaren Einrichtung (46) vorgesehen ist, und bei der das Ende derart ausgelegt ist, daß es die Fläche der Anbringungseinrichtung für das zweite Teil berührt, wenn die Teile ineinandergreifen.

10. Ostomievorrichtung nach Anspruch 9, bei der der Körperabschnitt (50) federnd nachgiebig ist und das Ende (52) in Berührung mit der Fläche vorbelastet, wenn die Teile ineinandergreifen.

11. Ostomievorrichtung nach Anspruch 1, die ferner eine zweite Einrichtung zum lösbaren Verbinden des Beutels und des Plättchens aufweist, wobei die zweite Verbindungseinrichtung erste und zweite ineinandergreifende Hilfsteile (50, 52), eine Einrichtung zum Anbringen eines (50) der ineinandergreifenden Hilfsteile an dem Beutel und eine Einrichtung zum Anbringen des anderen der ineinandergreifenden Hilfsteile (52) an dem Plättchen (86) aufweist.

12. Ostomievorrichtung nach Anspruch 11, bei der die ineinandergreifenden Hilfsteile einen Haken (58) und eine Öse (62) aufweisen.

13. Ostomievorrichtung nach Anspruch 11, bei der die ineinandergreifenden Hilfsteile einen Vorsprung (53) und eine Öffnung (56) aufweisen.

14. Ostomievorrichtung nach Anspruch 1, bei der die ineinandergreifenden Teile einen Vorsprung und eine Ausnehmung aufweisen.

15. Ostomievorrichtung nach Anspruch 1, bei der die ineinandergreifenden Teile ringförmig sind.

16. Ostomievorrichtung nach Anspruch 1, bei der die ineinandergreifenden Teile einen ringförmigen Vorsprung und eine ringförmige Ausnehmung aufweisen.

17. Ostomievorrichtung nach Anspruch 1, bei der das erste Teil einen ringförmigen Vorsprung und das zweite Teil eine ringförmige Ausnehmung aufweist.

18. Ostomievorrichtung nach Anspruch 1, bei der das erste Teil eine ringförmige Ausnehmung und das zweite Teil einen ringförmigen Vorsprung aufweist.

19. Ostomievorrichtung nach Anspruch 1, die ferner eine Wulst aufweist, die längs des inneren Randes des Abschnitts der Anbringungseinrichtung für das zweite Teil vorgesehen ist.

20. Ostomievorrichtung nach Anspruch 1, bei der der innere Rand des Abschnitts der Anbringungseinrichtung für das zweite Teil abgerundet ist.

21. Ostomievorrichtung nach Anspruch 1, die ferner eine Einrichtung (190) aufweist, die die dehnbare Einrichtung im gedehnten Zustand festlegt.

22. Ostomievorrichtung nach Anspruch 21, bei der die Halteeinrichtung ein Element aufweist, das zwischen der Anbringungseinrichtung für das zweite Teil und dem Plättchen anordenbar ist.

23. Ostomievorrichtung nach Anspruch 21, bei der die Halteeinrichtung abnehmbar ist.

24. Ostomievorrichtung nach Anspruch 21, bei der die Halteeinrichtung ein ringähnliches Element aufweist.

25. Ostomievorrichtung nach Anspruch 21, bei der die Halteeinrichtung aus elastischem Material besteht.

26. Ostomievorrichtung nach Anspruch 21, bei der die Halteeinrichtung aus einem geschlossenzelligen Schaum besteht.

27. Ostomievorrichtung nach Anspruch 21, bei der die Halteeinrichtung einen im wesentlichen kreisförmigen Querschnitt hat.

28. Ostomievorrichtung nach Anspruch 21, bei der die Halteeinrichtung einen aufrecht stehenden Abschnitt und einen Basisabschnitt aufweist.

29. Ostomievorrichtung nach Anspruch 1, bei der die ineinandergreifenden Teile eine längliche Form haben.

30. Ostomievorrichtung nach Anspruch 1, bei der die ineinandergreifenden Teile eine im wesentlichen ovale Gestalt haben.

## Revendications

1. Dispositif de stomie comprenant une poche (12), une étiquette (26) à dos adhésif et des moyens (20, 32) de liaison amovible entre ladite poche et ladite étiquette, lesdits moyens de liaison comprenant une première (20) et une seconde (32) parties à prise mutuelle, un moyen de montage de ladite première partie sur ladite poche et un moyen de montage de ladite seconde partie sur ladite étiquette, dans une position perpendiculaire à la surface de ladite étiquette, ledit moyen de montage de la seconde partie comprenant une section (40), espacée de ladite seconde partie, qui est conçue pour être fixée à la surface de ladite étiquette, caractérisé par un moyen extensible (46), interposé entre ladite section (40) et ladite seconde partie (32), pour faciliter le déplacement de ladite seconde partie (32) par rapport à ladite position perpendiculaire.

2. Dispositif selon la revendication 1, dans lequel ledit moyen de montage de la seconde partie comprend un élément, et dans lequel ledit moyen extensible (46) comprend un ou plusieurs plis en accordéon dans ledit élément.

3. Dispositif selon la revendication 1, dans lequel ledit moyen extensible (46) est dirigé sensiblement parallèlement à ladite surface de ladite étiquette (26).

4. Dispositif selon la revendication 1, dans lequel ledit moyen extensible est dirigé sensiblement perpendiculairement à ladite surface de ladite étiquette (26).

5. Dispositif selon la revendication 1, dans lequel ledit moyen de montage de la seconde partie comprend en outre une seconde section (33), appelée également ici "rebord", qui s'étend entre et réunit ledit moyen extensible (46) et ladite seconde partie (32).

6. Dispositif selon la revendication 5, dans lequel ladite seconde section est fixée à ladite seconde partie (32) en un point de la surface sous-jacente de ladite partie voisin du côté de ladite partie.

7. Dispositif selon la revendication 1, dans lequel ladite première partie (20) comprend en outre un moyen (48) conçu pour recouvrir ledit moyen extensible (46) quand lesdites première et seconde parties sont en prise mutuelle.

8. Dispositif selon la revendication 7, dans lequel ledit moyen de couverture comprend un élément (50) de type rebord, partant de ladite première partie.

9. Dispositif selon la revendication 8, dans lequel ledit élément comprend un corps et une extrémité libre (52), ledit corps (50) étant conçu pour se trouver dans l'alignement dudit moyen extensible (46) et ladite extrémité étant conçue pour venir au contact de la surface dudit moyen de montage de la seconde partie, quand lesdites parties viennent en prise mutuelle.

10. Dispositif selon la revendication 9, dans lequel ledit corps (50) est élastique et pousse ladite extrémité (52) en contact avec ladite surface, quand lesdites parties sont en prise mutuelle.

11. Dispositif selon la revendication 1, comprenant en outre un second moyen de liaison amovible entre ladite poche et ladite étiquette, ledit second moyen de liaison comprenant une première et une seconde parties auxiliaires à prise mutuelle (50, 52), un moyen de montage de l'une (50) desdites parties auxiliaires sur ladite poche et un moyen de montage de l'autre (52) desdites parties auxiliaires sur ladite étiquette (86).

12. Dispositif selon la revendication 11, dans lequel lesdites parties auxiliaires à prise mutuelle comprennent un crochet (58) et un oeillet (62).

13. Dispositif selon la revendication 11, dans lequel lesdites parties auxiliaires à prise mutuelle comprennent une saillie (53) et une ouverture (56).

14. Dispositif selon la revendication 1, dans lequel lesdites parties à prise mutuelle comprennent une saillie et une gorge.

15. Dispositif selon la revendication 1, dans lequel lesdites parties à prise mutuelle sont annulaires.

16. Dispositif selon la revendication 1, dans lequel lesdites parties à prise mutuelle comprennent une saillie en forme d'anneau et une gorge en forme d'anneau.

17. Dispositif selon la revendication 1, dans lequel ladite première partie comprend une saillie en forme d'anneau et ladite seconde partie comprend une gorge en forme d'anneau.

18. Dispositif selon la revendication 1, dans lequel ladite première partie comprend une gorge en forme d'anneau et ladite seconde partie comprend une saillie en forme d'anneau.

19. Dispositif selon la revendication 1, comprenant en outre une surépaisseur le long du bord intérieur de ladite section dudit moyen de montage de la seconde partie.

20. Dispositif selon la revendication 1, dans lequel le bord intérieur de ladite section dudit moyen de montage de la seconde partie est arrondi.

21. Dispositif selon la revendication 1, comprenant en outre un moyen (190) de retenue dudit moyen extensible dans son état dilaté.

22. Dispositif selon la revendication 21, dans lequel ledit moyen de retenue comprend un élément interposable entre ledit moyen de montage de la seconde partie et ladite étiquette.

23. Dispositif selon la revendication 21, dans lequel ledit moyen de retenue est détachable.

24. Dispositif selon la revendication 21, dans lequel ledit moyen de retenue comprend un élément du type bague.

25. Dispositif selon la revendication 21, dans lequel ledit moyen de retenue se compose d'un matériau élastique.

26. Dispositif selon la revendication 21, dans lequel ledit moyen de retenue se compose d'une mousse à cellules fermées.

27. Dispositif selon la revendication 21, dans lequel ledit moyen de retenue a une section droite sensiblement circulaire.

28. Dispositif selon la revendication 21, dans lequel ledit moyen de retenue comprend une partie verticale et une base.

29. Dispositif selon la revendication 1, dans lequel lesdites parties à prise mutuelle ont une forme allongée.

30. Dispositif selon la revendication 1, dans lequel lesdites parties à prise mutuelle ont une forme sensiblement ovale.

FIG. 1

FIG. 2

1

*FIG. 3*

*FIG. 4*

*FIG. 5*

# FIG. 6

# FIG. 7

FIG. 8

FIG. 9

0 098 718

F I G.10

F I G.11

5

# F I G. 12

# F I G. 13

## F I G.14

## F I G.15

F I G. 16

F I G. 17